# EUROPÄISCHE PATENTSCHRIFT

(11) **EP 1 903 939 B1**
(45) Veröffentlichungstag und Bekanntmachung des Hinweises auf die Patenterteilung: **14.08.2013**
(21) Anmeldenummer: 06754455.1
(22) Anmeldetag: 20.06.2006
(51) Int. Cl.: A61B 5/053, A61B 5/0245, A61B 5/0205, A61B 5/024, G01G 19/50

(54) **WAAGE MIT PULSMESSUNG**
SCALE WITH PULSE MEASUREMENT
BALANCE POURVUE D'UN SYSTEME DE MESURE DU POULS

(30) Priorität: 22.06.2005 DE 102005029189
(43) Veröffentlichungstag der Anmeldung: 02.04.2008
(73) Patentinhaber: Leifheit AG, 56377 Nassau/Lahn (DE)
(72) Erfinder: STAHL, Albrecht, 71560 Sulzbach (DE)
(74) Vertreter: Bungartz, Klaus Peter
(86) Internationale Anmeldenummer: PCT/EP2006/005901
(87) Internationale Veröffentlichungsnummer: WO 2006/136368

(56) Entgegenhaltungen:
- EP-A- 0 823 238
- EP-A- 1 125 550
- EP-A- 1 136 037
- EP-A1- 1 306 051
- EP-A2- 1 954 179
- DE-U1-202004 012 447
- DE-U1-202004 014 354

## Beschreibung

Die Erfindung betrifft eine Waage mit einer Vorrichtung zur Messung der Pulsfrequenz einer auf der Waage befindlichen Person nach dem Oberbegriff des Anspruchs 1.

Eine Waage nach dem Oberbegriff von Anspruch 1 ist aus DE 20 2004 012 447 U1 bekannt.

Aus DE 196 39 095 A1 ist bekannt, die Pulsfrequenz einer zu wiegenden Peron durch die pulsbedingten Gewichtsschwankungen zu ermitteln. Dies funktioniert nicht sehr zuverlässig.

Aufgabe der Erfindung ist es, eine Waage anzugeben, die auf zuverlässige Wiese die Pulsfrequenz der zu wiegenden Person ermittelt.

Die Aufgabe wird durch eine Waage gemäß Anspruch 1 gelöst.

Die Erfindung hat den Vorteil, dass eine zuverlässige Messung der Pulsfrequenz falls gewünscht während eines Wiegevorganges ermöglicht ist.

Erfindungsgemäß wurde erkannt, dass sich durch den zu- und abnehmenden Blutschwall die optischen Transmissivität und/oder der optischen Reflexivität der zu untersuchenden Person insbesondere im Fußbereich ändert.

Die Waage misst die Leistung des von der Peron ausgehenden Streulichte und/oder des von der Person ausgehenden Reflexionslichtes und/oder des von der Person ausgehenden Transmissionslichtes in Abhängigkeit von der Zeit.

Die erfindungsgemäße Waage ermittelt aus der gemessenen zeitlichen Abhängigkeit eine Pulsfrequenz und zeigt in einer Ausführungsform diese dem Benutzer auf einem Display an. Es kann auch vorgesehen sein, dass der Pulsschlag akustisch - beispielsweise durch Pieptöne - ausgegeben wird.

In eines Ausführungsform sind die Lichtquelle und der Lichtsensor benachbart zueinander derart angeordnet, dass sich die Abstrahlrichtung der Lichtquelle und die Empfangsrichtung des Lichtsensors kreuzen. Hierdurch ist eine sichere Messung in Reflexions- bzw. Streulichtanordnung ermöglicht. Eine Transmissionsanordnung ist prinzipiell auch denkbar, jedoch bei einer Personenwaage nicht mit annähernd gleichem Komfort für den Benutzer realisierbar, da eine Art Messgabel aufgebaut werden muss, in der Benutzer ein Fuß oder einen Teil des Fußes einführen muss; Während in Reflexions- bzw. Streulichtanordnung ein einfaches aufsteigen auf die Wiegefläche genügt.

In einer Ausführungsform beinhaltet das Messlicht Infrarotlicht.

In einer weiteren Ausführungsform der Waage weist die Lichtquelle eine LED (Light-Emitting-Diode) und/oder eine OLED (Organic-Light-Emitting-Diode) und/oder einen Laser - insbesondere einen Halbleiterlaser - auf.

In einer weiteren Ausführungsform der Waage ist der Lichtsensor als Photodiode und/oder als Photomultiplier und/oder CCD -Element ausgeführt.

Vorzugsweise sind die Lichtquelle und/oder der Lichtsensor und/oder die Vorrichtung zur Messung der Pulsfrequenz in die Auftrittfläche der Waage integriert.

In einer weiteren besonderen Ausführungsform der Waage sind die Lichtquelle und/oder der Lichtsensor in dem Bereich angeordnet, in dem ein Zeh - vorzugsweise ein großer Zeh - der zu wiegenden Peron beim Auftritt auf die Waage zu liegen kommt.

In einer anderen Ausführungsform der Waage sind die Lichtquelle und/oder der Lichtsensor in dem Bereich angeordnet sind, in dem eine Fußsohle - vorzugsweise der innere Längsbogen eine Fußsohle und/oder die Fußwölbung - der zu wiegenden Peron beim Auftritt auf die Waage zu liegen kommt. Diese Ausführungsform misst besonders zuverlässig und genau, weil die Haupt im Bereich der Fußsohle - insbesondere im Bereich der Fußwölbung und ganz besonders im Bereich des inneren Längsbogens - besonders dünn ist, so dass hier besonders gut gemessen werden kann.

In einer weiteren Ausführungsform ist eine Andrückvorrichtung vorgesehen, die die Lichtquelle und/oder den Lichtsensor gegen die zu wiegende Person drückt. Vorzugsweise erzeugt die Andrückvorrichtung eine zeitlich konstante Anpresskraft. Die Andrückvorrichtung kann federbeaufschlagt sein.

In einer besonderen Ausführung ist eine - beispielsweise mechanisch und/oder elektronisch arbeitende - Regelvorrichtung zur Regelung der Anpresskraft vorgesehen.

Durch die Ermittlung der Pulsfrequenz mit zwei verschiedenen Messmethoden - beispielsweise optisch und elektrisch - kann durch geeignete Verrechnung der verschiedenen Messsignale ein insgesamt sehr genaues Messergebnis erzielt werden.

Erfindungsgemäß ist nun zusätzlich weiterhin eine Vorrichtung zur zeitabhängigen Messung des elektrischen Körperwiderstandes einer auf der Waage befindlichen Person vorgesehen ist. Erfindungsgemäß wurde erkannt, dass sich durch den pulsbedingten zu- und abnehmenden Blutschwall beispielsweise in den Beinen der zu untersuchenden Person der - in der Regel komplexe - Körperwiderstand periodisch ändert.

Aus dieser zeitlichen Abhängigkeit des Körperwiderstand ermittelt die erfindungsgemäße Waage in einer vorteilhaften Ausführung - neben der optisch ermittelten - eine weitere Pulsfrequenz, die mit der optisch ermittelten Pulsfrequenz zu einer genaueren Pulsfrequenz verrechnet werden kann.

In einer vorteilhaften Ausführung ist vorgesehen, dass die Waage - zur Erzielung einer genaueren Pulsfrequenz - den gemessenen elektrischen Körperwiderstand mit den Signalen des Lichtsensors verrechnet.

Vorzugsweise weist die Waage - beispielsweise auf der Auftrittfläche - zumindest eine erste Elektrode und eine zweite Elektrode auf.

In einer Ausführungsform der Waage sind die erste Elektrode und/oder die zweite Elektrode in die Auftrittfläche der Waage integriert. In einer anderen Ausführungsform sind die erste Elektrode und/oder die zweite Elektrode auf der Auftrittfläche angeordnet.

In einer besonders vorteilhaften Ausführung sind - zur Herstellung eines besonders guten elektrischen Kontaktes - die erste Elektrode und/oder die zweite Elektrode und/oder die Auftrittfläche anschmiegsam ausgebildet. Hierdurch wird die Kontaktfläche zwischen den Füßen der zu untersuchenden Person und den Elektroden vergrößert. Diese Ausführungsform funktioniert auch bei Personen mit sehr trockenen Füßen sehr zuverlässig.

In einer vorteilhaften Ausführung weist die Waage eine durchsichtige Trittfläche auf. Hierbei kann vorteilhafter Weise vorgesehen sein, dass die erste Elektrode und/oder die zweite Elektrode eine leitende durchsichtige Beschichtung aufweist und/oder aus einer leitenden, durchsichtigen Beschichtung besteht. Der Benutzer kann so beispielsweise einen auf der Unterseite der Waage angebrachten Hinweis wahrnehmen.

Zur Messung des elektrischen Körperwiderstandes beihaltet die Waage vorzugsweise eine elektrische Spannungsquelle. In einer vorteilhaften Ausführung ist eine Spannungsmessvorrichtung und/oder eine Strommessvorrichtung vorgesehen.

In einer vorteilhaften Ausführung beinhaltet die Waage eine Auswertevorrichtung, die aus eingestellten und/oder gemessenen Spannungswerten und/oder Stromwerten zeitabhängig den Körperwiderstand ermittelt.

In einer vorteilhaften Ausführung ermittelt die Waage aus dem Körperwiderstand zusätzlich den Fettanteil und/oder den Wasseranteil des Körpers des Benutzers.

In der Zeichnung ist der Erfindungsgegenstand schematisch dargestellt und wird anhand der Figuren nachfolgend beschrieben, wobei gleich wirkende Elemente mit denselben Bezugszeichen versehen sind.

Dabei zeigen:
- Fig. 1: eine Waage ohne erfindungsgemäße Weiterbildung und
- Fig. 2: eine erfindungsgemäß ausgebildete Waage.

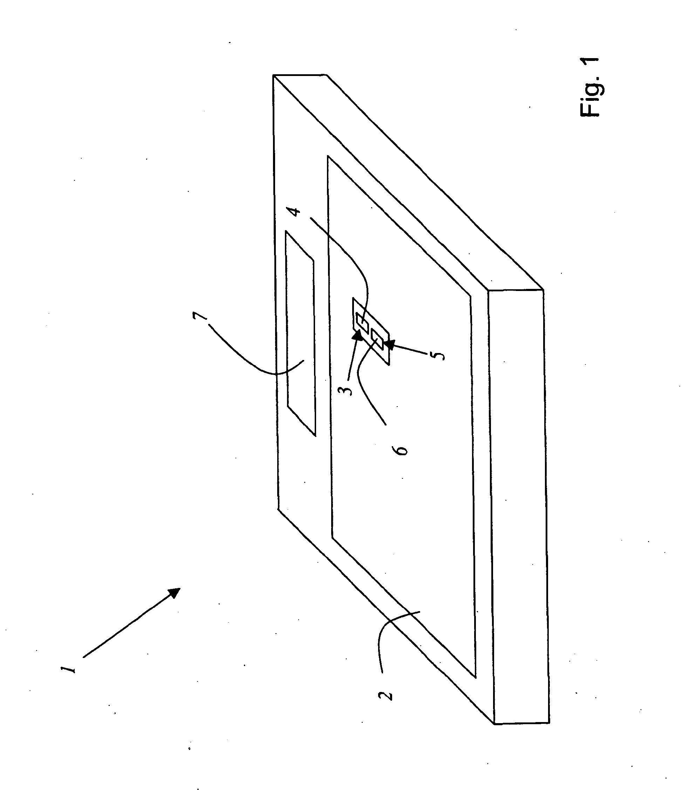

Fig. 1 zeigt eine Waage 1, die eine Lichtquelle 3 aufweist, die als Leuchtdiode 4 ausgebildet ist und Messlicht in Richtung auf die zu wiegende Person abgestrahlt. Neben der Lichtquelle 3 ist ein Lichtsensor 5, der als Photodiode 6 ausgebildet ist, zum Empfangen des von der Peron ausgehenden Streulichtes und/oder Reflexionslichtes und/oder Transmissionslichtes angeordnet.

Die Waage 1 misst das von der Person ausgehende Streulicht und/oder Reflexionslicht und/oder Transmissionslicht in Abhängigkeit von der Zeit und ermittelt aus den Messwerten eine Pulsfrequenz. Die Lichtquelle 3 und der Lichtsensor 5 sind in die Auftrittfläche 2 der Waage 1 integriert und in dem Bereich angeordnet, in dem ein Zeh - vorzugsweise ein großer Zeh - der zu wiegenden Peron beim Auftritt auf die Waage 1 zu liegen kommt. Die ermittelte Pulsfrequenz wird dem Benutzer auf einem Display 7 anzeigt.

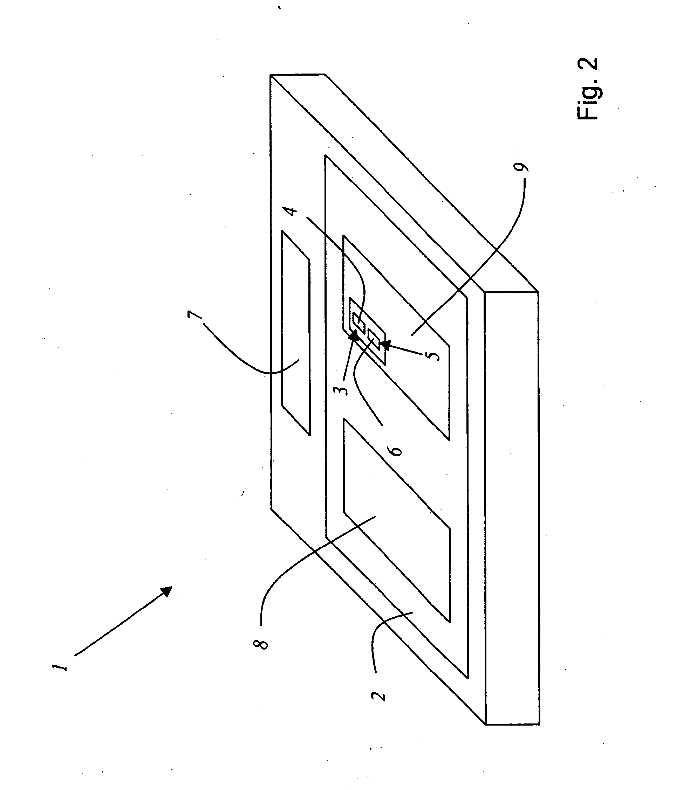

Fig. 2 zeigt eine erfindungsgemäße weitergebildete Waage 1 aus Basis der in Figur 1 gezeigten Waage. Die neue Waage ermittelt ebenso wie die in Fig. 1 dargestellte Waage 1 die Pulsfrequenz der auf der Waage 1 befindlichen Person durch Messung der zeitlichen Abhängigkeit des von der Peron ausgehenden Streulichtes und/oder Reflexionslichtes und/oder Transmissionslichtes. Zusätzlich ermittelt die Waage 1 nun durch Messung elektrischen Körperwiderstandes zwischen einer ersten Elektrode 8 und einer zweiten Elektrode 9 eine weitere Pulsfrequenz.

Die Waage 1 ist derart ausgebildet, dass die zu wiegende Person zur Durchführung einer Messung einen Fuß mit der ersten Elektrode 8 und den andern Fuß mit der zweiten Elektrode 9 in elektrischen Kontakt bringt. Die Waage 1 misst die Leistung des von der Peron ausgehenden Streulichtes und/oder Reflexionslichtes und/oder Transmissionslichtes in Abhängigkeit von der Zeit und ermittelt aus den Messwerten eine Pulsfrequenz, die anschließend mit der über den Körperwiderstand ermittelten weitere Pulsfrequenz verrechnet wird, um insgesamt ein genaues Ergebnis zu erhalten, das dem Benutzer auf einem Display 7 anzeigt wird.

### Bezugszeichenliste

- 1: Waage
- 2: Auftrittfläche
- 3: Lichtquelle
- 4: Leuchtdiode
- 5: Lichtsensor
- 6: Photodiode
- 7: Display
- 8: erste Elektrode
- 9: zweite Elektrode

## Patentansprüche

1. Waage (1) mit einer Vorrichtung zur Messung der Pulsfrequenz einer auf einer Auftrittfläche (2) der Waage (1) stehenden Person, wobei die Waage (1) eine Lichtquelle (3) aufweist, die Messlicht in Richtung auf die Person abgestrahlt, und wobei die Waage (1) einen Lichtsensor (5) zum Empfangen des von der Person ausgehenden Streulichtes und/oder Reflexionslichtes und/oder Transmissionslichtes aufweist, **dadurch gekennzeichnet, dass**
die Waage (1) die Pulsfrequenz aus der Abhängigkeit der Leistung des von der Person ausgehenden Streulichtes und/oder Reflexionslichtes und/oder Transmissionslichtes von der Zeit ermittelt und die Waage (1) eine Vorrichtung zur zeitabhängigen Messung des elektrischen Körperwiderstandes der auf der Waage (1) befindlichen Person aufweist, wobei zur Erzielung einer genaueren Pulsfrequenz die Waage (1) aus der Abhängigkeit des Körperwiderstandes von der Zeit eine weitere Pulsfrequenz ermittelt und die ermittelte weitere Pulsfrequenz mit der ermittelten Pulsfrequenz verrechnet und/oder die Waage (1) den gemessenen elektrischen Körperwiderstand mit den Signalen des Lichtsensors (5) verrechnet.

2. Waage (1) nach Anspruch 1, **dadurch gekennzeichnet, dass** das Messlicht Infrarotlicht beinhaltet.

3. Waage (1) nach einem der Ansprüche 1 oder 2, **dadurch gekennzeichnet, dass** die Lichtquelle (3) und/oder der Lichtsensor (5) in die Auftrittfläche (2) der Waage (1) integriert ist.

4. Waage (1) nach einem der Ansprüche 1 bis 3, **dadurch gekennzeichnet, dass** die Lichtquelle (3) und/oder der Lichtsensor (5) in dem Bereich angeordnet sind, in dem ein Zeh - vorzugsweise ein großer Zeh - der zu wiegenden Peron beim Auftritt auf die Waage (1) zu liegen kommt.

5. Waage (1) nach einem der Ansprüche 1 bis 3, **dadurch gekennzeichnet, dass** die Lichtquelle (3) und/oder der Lichtsensor (5) in dem Bereich angeordnet sind, in dem eine Fußsohle - vorzugsweise der innere Längsbogen eine Fußsohle - der zu wiegenden Peron beim Auftritt auf die Waage (1) zu liegen kommt.

6. Waage (1) nach einem der Ansprüche 1 bis 5, **dadurch gekennzeichnet, dass** eine Andrückvorrichtung vorgesehen ist, die die Lichtquelle (3) und/oder den Lichtsensor (5) gegen die zu wiegende Person drückt.

7. Waage (1) nach einem der Ansprüche 1 bis 6, **dadurch gekennzeichnet, dass** die Waage (1) die zeitliche Veränderung der optischen Transmissivität und/oder der optischen Reflexivität der zu wiegenden Person im Messbereich - vorzugsweise im Fußbereich - misst.

8. Waage (1) nach einem der Ansprüche 1 bis 7, **dadurch gekennzeichnet, dass** die Waage (1) - vorzugsweise auf der Auftrittfläche (2) - zumindest eine erste Elektrode (8) und eine zweite Elektrode (9) aufweist und dass die Vorrichtung zur Messung den elektrischen Körperwiderstand zwischen der erste Elektrode (8) und der zweiten Elektrode (9) misst.

9. Waage (1) nach einem der Ansprüche 1 bis 8, **dadurch gekennzeichnet, dass** die Waage (1) die Pulsfrequenz dem Benutzer anzeigt und/oder akustisch - insbesondere durch Pieptöne - ausgibt.

10. Waage (1) nach Anspruch 8 oder 9, **dadurch gekennzeichnet, dass** die Waage (1) eine durchsichtige Auftrittfläche (2) aufweist und dass die erste Elektrode (8) und/oder die zweite Elektrode (9) eine leitende, durchsichtige Beschichtung aufweist und/oder aus einer leitenden, durchsichtigen Beschichtung besteht.

11. Waage (1) nach einem der Ansprüche 1 bis 10, **dadurch gekennzeichnet, dass** die Waage (1) über eine Auswertevorrichtung aus eingestellten und/oder gemessenen Spannungswerten und/oder Stromwerten einen Körperwiderstand ermittelt.

## Claims

1. Scales (1) having a device for measuring the pulse rate of a person standing on a standing surface (2) of the scales (1), wherein the scales (1) have a light source (3) which emits measuring light in the direction of the person, and wherein the scales (1) have a light sensor (5) for receiving the scattered light and/or reflected light and/or transmitted light emitted by the person, **characterized in that**
the scales (1) determine the pulse rate from the dependence on time of the power of the scattered light and/or reflected light and/or transmitted light emitted by the person, and the scales (1) have a device for time-dependent measurement of the electric resistance of the body of the person standing on the scales (1), wherein to achieve a more accurate pulse rate, the scales (1) determine an additional pulse rate from the dependence of the body weight on time, and the additional pulse rate thereby determined is calculated with the pulse rate determined, and/or the scales (1) calculate the measured electric resistance of the person's body using the signals of the light sensor (5).

2. The scales (1) according to Claim 1, **characterized in that** the measuring light includes infrared light.

3. The scales (1) according to any one of Claims 1 or 2, **characterized in that** the light source (3) and/or the light sensor (5) is/are integrated into the standing surface (2) of the scales (1).

4. The scales (1) according to any one of Claims 1 to 3, **characterized in that** the light source (3) and/or the light sensor (5) is/are arranged in the area in which a toe - preferably a big toe - of the person being weighed comes to lie on the scales (1) when stepping on them.

5. The scales (1) according to any one of Claims 1 to 3, **characterized in that** the light source (3) and/or the light sensor (5) is/are arranged in the area in which the sole of a person's foot - preferably the inner arch of the sole of a foot of the person being weighed - comes to rest on the scales (1) when the person steps on the scales.

6. The scales (1) according to any one of Claims 1 to 5, **characterized in that** a pressing device which presses the light source (3) and/or the light sensor (5) against the person to be weighed is provided.

7. The scales (1) according to any one of Claims 1 to 6, **characterized in that** the scales (1) measure the change in the optical transmissivity and/or the optical reflexivity over time of the person being weighed in the measurement range - preferably in the foot area.

8. The scales (1) according to any one of Claims 1 to 7, **characterized in that** the scales (1) have at least one first electrode (8) and one second electrode (9) - preferably on the standing surface (2) - and the device for measuring the electric resistance of the body performs the measurement between the first electrode (8) and the second electrode (9).

9. The scales (1) according to any one of Claims 1 to 8, **characterized in that** the scales (1) display the pulse rate for the user and/or output it acoustically - in particular through beeping sounds.

10. The scales (1) according to Claim 8 or 9, **characterized in that** the scales (1) have a transparent standing surface (2) and the first electrode (8) and/or the second electrode (9) has/have a conductive transparent coating and/or consist(s) of a conductive transparent coating.

11. The scales (1) according to any one of Claims 1 through 10, **characterized in that** the scales (1) determine the resistance of a person's body via an evaluation device from set and/or measured voltage values and/or current values.

## Revendications

1. Balance (1) avec un dispositif pour la mesure de la fréquence de pouls d'une personne debout sur une surface d'appui (2) de la balance (1), où la balance (1) comporte une source de lumière (3) émettant une lumière de mesure en direction de la personne, et où la balance (1) comporte un capteur de lumière (5) destiné à recevoir la lumière diffusée et/ou la lumière réfléchie et/ou la lumière transmise par la personne, **caractérisée en ce que**
la balance (1) détermine la fréquence de pouls à partir de la dépendance de la puissance de la lumière diffusée et/ou de la lumière réfléchie et/ou de la lumière transmise par la personne à l'égard du temps, et la balance (1) comporte un dispositif pour la mesure en fonction du temps de la résistance corporelle électrique de la personne sur la balance (1), dans laquelle, pour obtenir une fréquence de pouls plus précise, la balance (1) détermine une nouvelle fréquence de pouls à partir de la dépendance de la résistance corporelle à l'égard du temps, et calcule la nouvelle fréquence de pouls déterminée avec la fréquence de pouls déterminée et/ou la balance (1) calcule la résistance corporelle mesurée avec les signaux du capteur de lumière (5).

2. Balance (1) selon la revendication 1, **caractérisée en ce que** la lumière de mesure contient une lumière infrarouge.

3. Balance (1) selon l'une des revendications 1 ou 2, **caractérisée en ce que** la source de lumière (3) et/ou le capteur de lumière (5) sont intégrés dans la surface d'appui (2) de la balance (1).

4. Balance (1) selon l'une des revendications 1 à 3, **caractérisée en ce que** la source de lumière (3) et/ou le capteur de lumière (5) sont agencés dans la région sur laquelle un orteil - de préférence le gros orteil - de la personne à peser s'applique lorsque celle-ci monte sur la balance (1).

5. Balance (1) selon l'une des revendications 1 à 3, **caractérisée en ce que** la source de lumière (3) et/ou le capteur de lumière (5) sont agencés dans la région dans laquelle une plante de pied - de préférence l'arc longitudinal intérieur d'une plante de pied - de la personne à peser s'applique lorsque celle-ci monte sur la balance (1).

6. Balance (1) selon l'une des revendications 1 à 5, **caractérisée en ce qu'**il est prévu un dispositif de pression serrant la source de lumière (3) et/ou le capteur de lumière (5) contre la personne à peser.

7. Balance (1) selon l'une des revendications 1 à 6, **caractérisée en ce que** la balance (1) mesure la modification temporelle de la capacité de transmission optique et/ou de la capacité de réflexion optique de la personne à peser dans la région de mesure - de préférence dans la région du pied.

8. Balance (1) selon l'une des revendications 1 à 7, **caractérisée en ce que** la balance (1) - de préférence sur la surface d'appui (2) - comporte au moins une première électrode (8) et une deuxième électrode (9), et **en ce que** le dispositif pour la mesure de la résistance corporelle électrique mesure entre la première électrode (8) et la deuxième électrode (9).

9. Balance (1) selon l'une des revendications 1 à 8, **caractérisée en ce que** la balance (1) affiche et/ou émet de façon acoustique - en particulier par un signal sonore - la fréquence de pouls à l'utilisateur.

10. Balance (1) selon la revendication 8 ou 9, **caractérisée en ce que** la balance (1) comporte une surface d'appui transparente (2) et **en ce que** la première électrode (8) et/ou la deuxième électrode (9) comporte un revêtement transparent et conducteur et/ou est constituée d'un revêtement transparent et conducteur.

11. Balance (1) selon l'une des revendications 1 à 10, **caractérisée en ce que** la balance (1) détecte une résistance corporelle par le biais d'un dispositif d'évaluation, à partir de valeurs de tension et/ou de valeurs de courant réglées et/ou mesurées.
